# EUROPÄISCHE PATENTSCHRIFT

(11) **EP 3 597 826 B1**
(45) Veröffentlichungstag und Bekanntmachung des Hinweises auf die Patenterteilung: **02.09.2020**
(21) Anmeldenummer: 18184705.4
(22) Anmeldetag: 20.07.2018
(51) Int. Cl.: E01C 19/48, E01C 19/28, E01C 23/01, G01N 33/42, G01N 25/72, G01J 5/00

(54) **VERFAHREN UND STRASSENFERTIGER ZUM ERFASSEN VON STÖROBJEKTEN BEI DER ERSTELLUNG EINES TEMPERATURFELDS DER EINBAUSCHICHT**
METHOD AND PAVING MACHINE FOR DETECTING INTERFERENCE OBJECTS DURING THE CREATION OF A TEMPERATURE FIELD OF THE PAVING LAYER
PROCÉDÉ ET FINISSEUSE DE ROUTE POUR DÉTECTER DES OBSTACLES LORS DE LA RÉALISATION D'UN CHAMP DE TEMPÉRATURE DE LA COUCHE À POSER

(43) Veröffentlichungstag der Anmeldung: 22.01.2020
(73) Patentinhaber: Joseph Vögele AG, 67067 Ludwigshafen (DE)
(72) Erfinder: BUSCHMANN, Martin, 67435 Neustadt (DE); HENNING, Delius, 67480 Edenkoben (DE)
(74) Vertreter: Grünecker Patent- und Rechtsanwälte PartG mbB

(56) Entgegenhaltungen:
- EP-A1- 2 982 951

## Beschreibung

Die Erfindung bezieht sich auf ein Verfahren zum Erstellen eines Temperaturfelds gemäß dem Anspruch 1 sowie auf einen Straßenfertiger gemäß dem unabhängigen Anspruch 14.

Die Verarbeitungstemperatur von Asphalt ist eine wesentliche Prozessgröße im Asphaltstraßenbau und hat erheblichen Einfluss auf die Gebrauchseigenschaften, insbesondere auf die Lastfestigkeit, den Schichtenverbund sowie die Lebensdauer des eingebauten Belags. Aus diesem Grund gewinnt das Messen der Einbautemperatur neu verlegter Straßenbeläge zusehends an Bedeutung. Es gehört mittlerweile auch zur gängigen Praxis, dass Baulastenträger in zunehmendem Maße messtechnische Nachweise über die Einbautemperaturen verlangen.

Problematisch ist jedoch, dass es während des Einbaus bei der Bestimmung der Temperatur des aufgebrachten Straßenbelags immer wieder zu Fehlmessungen kommt, welche unter anderem durch Störobjekte hervorgerufen werden, wie z.B. durch Baustellenpersonal, Walzen oder Messeinheiten, die sich zumindest temporär im Messbereich hinter der Einbaubohle auf dem neuen Straßenbelag aufhalten und dadurch manche Messstellen des Temperaturfelds hinter der Einbaubohle verdecken. Wird dann eine bildliche Aufnahme deren Eigentemperatur im zu erzeugenden Temperaturfeld durchgeführt, wird das Temperaturfeld diesbezüglich zumindest stellenweise verfälscht, weil die Eigentemperatur solcher temporären Störobjekte deutlich außerhalb eines zu erwartenden Temperaturbereichs liegt. Insbesondere für Dokumentationszwecke ist ein solcher Zustand daher nicht zufriedenstellend.

Bisher werden zum Messen der Einbautemperatur des neu verlegten Straßenbelags hinter der Einbaubohle des Straßenfertigers unterschiedliche Messsysteme eingesetzt, beispielsweise Infrarotscanner sowie hin und her schwenkbare Pyrometereinheiten oder mehrere Pyrometer (Pyrometerarray). Mittels solcher Vorrichtungen lässt sich ein mehr oder weniger geschlossenes Temperaturabbild in Form eines Temperaturprofils der verlegten Einbauschicht erzeugen.

Ein bekanntes System verwendet dafür eine Infrarotkamera, deren Bilddaten durch eine Software zu Abtastzeilen verrechnet werden. Die Abtastzeilen haben einen definierten Abstand zu einer Hinterkante der Einbaubohle. Jede Abtastzeile repräsentiert einen transversalen Temperaturverlauf der eingebauten Schicht.

Zum Herstellen eines Abbildes des homogeneren Temperaturfeldverlaufs, d.h. ohne bildliche Darstellung vereinzelter Kühlstellen, werden in der Praxis auch Systeme eingesetzt, die dazu konfiguriert sind, in einem dem eigentlichen Temperaturmessprozess nachgelagerten Steuervorgang mittels Interpolation sollwertiger Temperaturmessungen dazwischenliegende nichtsollwertige Temperaturmessungen (Kühlstellen) zu überschreiben. Nachteilig daran ist allerdings, dass bei diesem mittels Interpolation durchgeführten Verfahren auch Messwerte überschrieben werden können, die tatsächlich auf zu kaltes Einbaumaterial, und somit nicht gezielt lediglich temporär auf der eingebauten Schicht positionierte Störobjekte erfassbar und im Temperaturfeld kompensiert werden.

Die WO 00/70150 A1 offenbart einen Straßenfertiger mit einer daran befestigten Erfassungseinheit zum Durchführen einer Temperaturerfassung der vom Straßenfertiger neu verlegten Einbauschicht, wobei gegebenenfalls erfasste Kühlstellen mittels einer Markiervorrichtung des Straßenfertigers für einen Verdichtungsvorgang gekennzeichnet werden.

Die DE 10 2014 222 693 A1 offenbart einen Straßenfertiger mit einer daran montierten Vorrichtung zur Bestimmung der Temperatur eines Straßenbelagmaterials, wobei die Vorrichtung einen Infrarottemperaturmesskopf aufweist, der anhand eines damit verbundenen Motors quer zur Fahrtrichtung des Straßenfertigers schwenkbar angeordnet ist.

Die EP 2 982 951 A1 offenbart einen Straßenfertiger mit einem am Dach positionierten Thermographiemodul, welches dazu ausgebildet ist, einen Abweichungsbereich des eingebauten Belags zu erkennen, sofern basierend auf für den Bereich erfassten Temperaturwerten ein oder mehrere Abweichungskriterien erfüllt sind, wobei dem Abweichungsbereich Bildaufnahmen, beispielsweise bezüglich temporärer Störobjekte, zugeordnet werden können.

Die EP 2 990 531 A1 offenbart ein System für Straßenfertiger mit einer Temperaturmessvorrichtung, anhand dessen durch wiederholtes Erfassen von Temperaturwerten eines von einem Straßenfertiger eingebauten Belages ein Abkühlverhalten des eingebauten Belages bestimmt werden kann.

Die EP 2 666 908 A1 offenbart ein Verfahren zum Bestimmen eines materialspezifischen Abkühlverhaltens für Materialmischungen, die im Straßenbau eingesetzt werden.

Die EP 2 789 741 A1 offenbart einen Straßenfertiger mit einer daran befestigten Thermographievorrichtung zum Erfassen eines Temperaturprofils mindestens eines Bereichs einer verlegten Einbauschicht hinter der Einbaubohle.

Um die tatsächliche Qualität einer Einbauschicht anhand eines Temperaturfelds entlang der Einbaustrecke besser beurteilen zu können, bzw. um damit eine verbesserte elektronische Auswertung und/oder ggf. Weiterverarbeitung vorzunehmen, ist es unabdingbar, dass keine, oder zumindest vernachlässigbar wenige Fehlmessungen im Messbereich des Temperaturfelds vorhanden sind, insbesondere Fehlmessungen die wegen temporärer Störobjekte im Temperaturfeld eingebunden sind.

Aufgabe der Erfindung ist es, ein Verfahren sowie einen Straßenfertiger zur Verfügung zu stellen, womit sich insbesondere in Abgrenzung zu tatsächlichen Materialfehlern der Einbauschicht gezielt Fehlmessungen hinsichtlich temporär auf der neuen Einbauschicht befindlicher Störobjekte erkennen lassen, damit ein um störobjektbezogene Fehlmessungen bereinigtes Temperaturfeld erzeugt werden kann.

Die Aufgabe der Erfindung wird gelöst mittels eines Verfahrens gemäß dem Anspruch 1 sowie mittels eines Straßenfertigers gemäß dem unabhängigen Anspruch 14.

Vorteilhafte Weiterbildungen der Erfindung sind in den Unteransprüchen angegeben.

Die Erfindung betrifft ein Verfahren zum Erstellen eines Temperaturfelds mindestens eines Abschnitts einer mittels einer Einbaubohle eines Straßenfertigers verlegten Einbauschicht, wobei ein temporär auf der verlegten Einbauschicht, innerhalb mindestens eines darauf vorbestimmten Messbereichs des Temperaturfelds positioniertes Störobjekt detektiert wird, dessen Eigentemperatur unterhalb eines gewünschten, sollwertigen Temperaturbereichs für den Messbereich liegt, sodass eine Abbildung der Eigentemperatur des Störobjekts im zu erzeugenden Temperaturfeld dasselbe zumindest stellenweise verfälscht, wobei zum Detektieren des Störobjekts eine Temperatur mindestens einer innerhalb des Messbereichs liegenden Messstelle zu unterschiedlichen Zeitpunkten gemessen wird.

Erfindungsgemäß wird erkannt, dass beim Verbau der Einbauschicht die Messstelle zu einem ersten Zeitpunkt von einem Störobjekt bedeckt wird, wenn entgegen einer zu erwartenden materialspezifischen Abkühlung zu einem späteren zweiten Zeitpunkt während des Verbaus an derselben Messstelle eine größerer, insbesondere innerhalb des sollwertigen Temperaturbereichs liegender, Temperaturwert als ein zu dem vorausgehenden ersten Zeitpunkt erfasster Temperaturwert gemessen wird, wobei anstelle des zum ersten Zeitpunkts erfassten Temperaturwerts ein neuer Temperaturwert der Messstelle im Temperaturfeld zugeordnet wird.

Im Rahmen der Erfindung ist als Temperaturfeld eine zweidimensionale Temperaturverteilung gemeint, anhand derer jeder Messstelle (Raumpunkt) eine Temperatur zugeordnet wird. Im Temperaturfeld können bestimmte Temperaturen an den jeweiligen Messstellen durch bestimmte Farben dargestellt sein. Bei der Erfindung bildet das Temperaturfeld vorzugsweise ein Wärmeabbild der Temperaturverteilung auf der Oberfläche der neu verlegten Einbauschicht.

Erfindungsgemäß wird somit mittels eines für eine Messstelle erfassten Temperaturgradienten auf ein Störobjekt an der Messstelle im Temperaturfeld hingewiesen, welches dort zumindest temporär zwischen den Messzeitpunkten positioniert war und dadurch die eigentlich beabsichtigte Temperaturmessung am dort eingebauten Deckenmaterial verhinderte. Der Temperaturgradient bietet eine einfache Messgröße, um das Vorliegen eines Störobjekts zuverlässig zu erfassen.

Demzufolge kann im Temperaturfeld mithilfe der Erfindung jedem Raumpunkt des Messbereichs eine tatsächlich dort gemessene Einbautemperatur zugeordnet werden, selbst dann, wenn die Erfassung der dortigen Einbautemperatur temporär durch ein Störobjekt behindert wurde. In anderen Worten können mittels der Erfindung störobjektbezogene Temperaturwerte zuverlässig bei der Herstellung des Temperaturfelds herausgefiltert werden.

Das Erfassen der Temperatur jeweiliger Messstellen läuft vorzugsweise während des Einbaus in wiederkehrenden Messintervallen ab. Dabei können die Messintervalle in Abhängigkeit der Einbaugeschwindigkeit gesteuert werden, sodass die Temperaturmessung an den jeweiligen Messstellen mit ausreichenden Wiederholungen stattfinden kann, um zuverlässig Störobjekte zu erfassen. Das erfindungsgemäße Verfahren kann damit fortlaufend Temperaturmessungen an den jeweiligen Messstellen durchführen, Störobjekte erfassen und diese bei der Temperaturfelderzeugung zuverlässig herausfiltern.

Als mittels der Erfindung erfassbares Störobjekt kommt beispielsweise ein Walzenfahrzeug in Frage, welches dem Straßenfertiger folgt, um die mittels des Straßenfertigers neu verlegte Einbauschicht auf ein vorbestimmtes Maß zu verdichten. Ferner können Messeinheiten, beispielsweise Nivelliergeräte, zumindest temporär auf dem neuen Straßenbelag innerhalb des Messbereichs positioniert sein, die die Temperaturfelderzeugung stören. Als Störobjekt kann auch Bedienpersonal detektiert werden, welches sich im Messbereich aufhält oder beim Durchqueren des Messbereichs die Temperaturfeldmessung stört. Grundsätzlich können als Störobjekte sämtliche Körper detektiert werden, die nicht Bestandteil der neuen Einbauschicht sind und eine unmittelbare Temperaturerfassung an der gewünschten Messstelle verhindern.

Bei der Erfindung lassen sich nicht plausible störobjektbezogene Messwerte unter Einsatz einfacher Verfahrensschritte, insbesondere unter Anwendung von ohnehin am Straßenfertiger vorgesehener Technik, erkennen, worauf basierend eine Korrektur des Temperaturfelds dahingehend durchgeführt werden kann, dass darin störobjektbezogene Fehlmessungen eliminiert bzw. zumindest stark reduziert werden können, wodurch das Temperaturfeld insgesamt, vor allem für Zwecke der Qualitätskontrolle, aussagekräftiger wird.

Hinzu kommt, dass das mittels der Erfindung um störobjektbezogene Fehlmessungen bereinigte Temperaturfeld besser als Steuereingangsgröße für Steuerungsprozesse, beispielsweise für Ablaufsteuerungen bestimmter Einbaufunktionen, am Straßenfertiger selbst und/oder an anderen am Einbau beteiligten Fahrzeugen oder Gerätschaften, beispielsweise an Materialvermischungsanlagen, an Materialbelieferungsfahrzeugen und/oder an Verdichtungsfahrzeugen, verwendet werden kann, um diesbezüglich daran durchgeführte Arbeitsprozesse optimal zu steuern.

Schließlich bietet das erfindungsgemäße Verfahren den technischen Vorteil, dass das damit hinsichtlich störobjektbezogener Fehlmessungen bereinigte Temperaturfeld gegenüber bekannten Lösungen ein reduziertes Datenspeichervolumen aufweist, sodass es besser dokumentier- und weiterverarbeitbar ist.

Eine Variante der Erfindung sieht vor, dass zum Detektieren des Störobjekts eine Objekterkennung in einer Auswertungssoftware zum Einsatz kommt. Vorzugsweise wird dabei zum Detektieren des Störobjekts in der Auswertungssoftware überprüft ob sich Objekte relativ zur Einbaugeschwindigkeit bewegen.

Vorzugsweise wird bei der Erstellung des Temperaturfelds für die neue Einbauschicht als neuer Temperaturwert, sozusagen als Korrekturwert, für die Messstelle der spätere, an derselben Messstelle erfasste größere Temperaturwert eingesetzt. Dies kann unter der Maßgabe geschehen, dass der größere Temperaturwert noch innerhalb des oder eines angepassten sollwertigen Temperaturbereichs liegt.

Steuerungstechnisch lässt sich dies beispielsweise dadurch verwirklichen, indem der zum ersten Zeitpunkt der Temperaturmessung wegen Vorliegens eines Störobjekts gemessene zu kalte Temperaturwert vom später erfassten, eigentlichen an derselben Messstelle vorliegenden größeren Temperaturwert von einer Steuereinheit überschrieben wird. Dies kann mittels eines Ablaufsteuervorgangs durchgeführt werden, wobei die Störobjekttemperatur an der Messstelle solange im Temperaturfeld gesetzt bleibt, bis eine größere Temperatur für die Messstelle detektiert wird. Dadurch ist es möglich, dass eine ggf. zunächst fälschlicherweise wegen des Vorliegens eines Störobjekts einer Messstelle zugeordnete Störobjekt-Eigentemperatur im Nachhinein, insbesondere mittels einer Rückkopplungssteuerung, während des Verbaus durch die an der Messstelle eigentlich vorliegende Einbautemperatur des Einbaumaterials ersetzt wird, sozusagen zum späteren Zeitpunkt für dieselbe Messstelle eine Korrektur hinsichtlich des neuen Temperaturwerts durchgeführt wird.

Besser an die tatsächliche Einbausituation lässt sich das Verfahren dadurch anpassen, indem der spätere, an derselben Messstelle erfasste größere Temperaturwert noch unter Berücksichtigung eines vorbestimmten oder ermittelten Abkühlkoeffizienten mindestens einer benachbarten Messstelle korrigiert wird, bevor der dadurch gewonnene neue Temperaturwert den zum ersten Zeitpunkt erfassten Temperaturwert ersetzt. Damit lässt sich eine Abkühlung an der Messstelle berücksichtigen, sodass es keine Rolle spielt, wie lange das Störobjekt an der Messstelle positioniert war, weil sich anhand des Abkühlkoeffizienten exakt ermitteln lässt, wie die eigentliche Temperatur der Messstelle tatsächlich zum vorausgehenden ersten Zeitpunkt im Temperaturfeld ausgefallen wäre, wenn das Störobjekt zu dieser Zeit nicht an der Messstelle positioniert gewesen wäre.

Eine Variante sieht vor, dass der neue Temperaturwert, vorzugsweise in Abhängigkeit des erfassten Temperaturgradienten an der Messstelle x, mithilfe eines Kennfelds berechnet wird.

Eine Ausführungsvariante der Erfindung sieht als Plausibilitätscheck vor, dass bei der Erstellung des Temperaturfelds für die Einbauschicht der zum ersten Zeitpunkt erfasste Temperaturwert durch den neuen Temperaturwert ersetzt wird, wenn der zum ersten Zeitpunkt erfasste Temperaturwert einen Temperaturschwellenwert nicht übersteigt. Dadurch kann sichergestellt werden, dass auch lediglich störobjektbezogene Fehlmessungen, deren Temperaturwerte unterhalb des Temperaturschwellenwerts liegen, korrigiert werden, also nicht etwa tatsächliche Fehlermessungen am Einbaumaterial der Einbauschicht, die auf stellenweise zu kalt verbautes Einbaumaterial hinweisen.

Der Einsatz des Temperaturschwellenwerts ermöglicht es auf einfachem Wege, verfahrenstechnisch störobjektbezogene Fehlmessungen eindeutig von einbaumaterialspezifischen Fehlermessungen, d.h. Kaltstellen in der Einbauschicht, zu unterscheiden, worauf basierend störobjektbezogene Fehlmessungen präzise im Temperaturfeld herausgefiltert werden können. Dagegen bleiben die typischerweise oberhalb des Temperaturschwellenwerts erfassten, jedoch nicht sollwertigen Temperaturfehlermessungen im Temperaturfeld enthalten, sodass das Temperaturfeld zuverlässig Einbaufehler anzeigt, sprich auf stellenweise entlang der Einbauschicht zu kalt verbautes Einbaumaterial hindeutet. Vorzugsweise ist der Temperaturschwellenwert in Abhängigkeit einer am Straßenfertiger erfassten Umgebungstemperatur und/oder angesichts einer für die Messstelle abgelaufenen Messzeit anpassbar.

Insbesondere praktikabel ist es, wenn jeweilige Temperaturwerte mehrerer Messstellen entlang mindestens einer quer zur oder in Einbaurichtung verlaufenden Messzeile innerhalb des Messbereichs erfasst werden. Vorzugsweise lässt sich die Auflösung entlang der Messzeile ändern, sodass in vorteilhafter Weise eine Abtastgenauigkeit angesichts unterschiedlicher Qualitätsanforderungen anpassbar ist.

Eine zeitversetzte Temperaturerfassung jeweiliger Messstellen ist insbesondere dadurch möglich, dass die mindestens eine Messzeile während es Verbaus der Einbauschicht zum Erfassen der jeweiligen Temperaturen an darin angeordneten Messstellen zu unterschiedlichen Zeitpunkten zumindest zeitweise entgegengesetzt zur Einbaurichtung bewegt wird. Während sich der Straßenfertiger bei der Einbaufahrt langsam in Einbaurichtung bewegt, wandert die Messzeile währenddessen, vorzugsweise von einer Einbaubohlenhinterkante ausgehend, langsam entlang einer vorbestimmten Messfeldlänge entgegen der Einbaurichtung nach hinten durch den Temperaturmessfeldbereich. Es ist vorstellbar, dass der Temperaturmessfeldbereich immer wieder aufs Neue mittels intervallweise durchgeführter Line-Scanbewegungen abgetastet wird, um auf der Einbauschicht positionierte Störobjekte zu detektieren. Für die Line-Scanbewegungen kann ein mittels eines Motors betriebener Zeilensensor eingesetzt werden.

In einer bevorzugten Ausführung ist der Einsatz einer IR-Kamera vorgesehen, da mit dieser mehrere Scan-Lines in ein aufgenommenes Bild gelegt werden können.

In einer bevorzugten Ausführungsvariante ist vorgesehen, dass ein Bewegungsablauf mindestens einer Messzeile in Abhängigkeit eines Einbauprozessparameters, insbesondere angesichts einer Einbaugeschwindigkeit des Straßenfertigers, gesteuert wird. Insbesondere ist es vorstellbar, dass in Abhängigkeit der erfassten Einbaugeschwindigkeit des Straßenfertigers automatisch, insbesondere proportional zur Einbaugeschwindigkeit, eine Abtastrate für einen Bewegungsablauf der Messzeile gesteuert wird. Dabei kann die Messzeile den Messbereich mit einer Abtastgeschwindigkeit durchlaufen, die einem Vielfachen der Einbaugeschwindigkeit entspricht, um das Vorhandensein von Störobjekten zuverlässig erfassen zu können.

Es ist auch vorstellbar, dass in Abhängigkeit der erfassten Einbaugeschwindigkeit des Straßenfertigers ein vorbestimmtes, daran geknüpftes Bewegungsschema für die Messzeile automatisiert abgerufen wird, wodurch der Messprozess optimal an den Einbaumodus des Straßenfertigers anpasst wird.

Eine Ausführungsform der Erfindung sieht vor, dass entlang einer Vielzahl von hintereinander quer zur Einbaurichtung jeweils in einen vorbestimmten Abstand relativ zu einer Bohlenhinterkante der Einbaubohle angeordnete Messzeilen die jeweiligen Temperaturen der darin angeordneten Messstellen gleichzeitig, beispielsweise mithilfe einer am Straßenfertiger befestigten IR-Kamera, während des Verbaus der Einbauschicht erfasst werden. Dies ermöglicht eine zweidimensionale Temperaturerfassung hinter der Einbaubohle an der Oberfläche der Einbauschicht.

Hinsichtlich unterschiedlicher Straßenfertigertypen, insbesondere hinsichtlich verschiedener Einbaubohlenbreiten, ist es besonders vorteilhaft, wenn eine quer zur Fahrtrichtung verlaufende Messzeilenbreite in Abhängigkeit einer Einbaubreite der Einbauschicht manuell und/oder automatisch angepasst werden kann.

Für die Praxis kann es vorteilhaft sein, wenn der sollwertige Temperaturbereich mindestens einer Messstelle, insbesondere für in einer Messzeile angeordnete Messstellen, in Abhängigkeit der verstrichenen Einbauzeit angepasst wird. Dies kann unter Berücksichtigung eines ermittelten Abkühlkoeffizienten mindestens einer benachbarten Messstelle geschehen. Damit ist es möglich, selbst nach einem längeren Verweilen eines Störobjekts an der Messstelle auf der Einbauschicht zu einem späteren Zeitpunkt eine dann dort bereits stark abgekühlte, angesichts eines ursprünglich für die Messstelle definierten sollwertigen Temperaturbereichs erfasste Einbaumaterialtemperatur rückwirkend noch in Zusammenhang mit einer störobjektbezogenen Fehlmessung zu bringen, worauf basierend der ursprünglich zum ersten Zeitpunkt fälschlicherweise erfasste Temperaturwert im Nachhinein korrigiert werden könnte. Tendenziell kann dabei mit zunehmend abgelaufener Einbauzeit der sollwertige Temperaturbereich niedrigere Temperaturwerte umfassen als zu vorangehenden Zeitpunkten während des Einbaus.

Vorzugsweise ist vorgesehen, dass, wenn ein Störobjekt erkannt wurde, am Straßenfertiger, am Störobjekt selbst und/oder an einem anderen am Einbau beteiligten Fahrzeug eine visuelle, akustische und/oder haptische Signalgebung einsetzt. Damit wird das am Einbauort anwesende Bedienpersonal auf die Herstellung des Temperaturfelds hingewiesen, und diesem insbesondere angezeigt, dass das erfasste Störobjekt aus dem Temperaturmessbereich herausbewegt werden soll.

Die Erfindung bezieht sich auch auf einen Straßenfertiger, umfassend eine Einbaubohle zum Verlegen einer Einbauschicht, eine Erfassungseinheit zum Erstellen eines Temperaturfelds mindestens eines Abschnitts der Einbauschicht im verlegten Zustand hinter der Einbaubohle sowie eine Steuereinheit, die dazu konfiguriert ist, basierend auf Messwerten der Erfassungseinheit ein temporär auf der Einbauschicht, innerhalb mindestens eines darauf vorbestimmten Messbereichs des Temperaturfelds positioniertes Störobjekt zu erkennen, dessen Eigentemperatur unterhalb eines gewünschten, sollwertigen Temperaturbereichs für den Messbereich liegt, sodass eine Abbildung der Eigentemperatur des Störobjekts im zu erzeugenden Temperaturfeld dasselbe zumindest stellenweise verfälscht.

Erfindungsgemäß ist die Erfassungseinheit dazu ausgebildet, zum Detektieren des Störobjekts eine Temperatur mindestens einer innerhalb des Messbereichs liegenden Messstelle zu unterschiedlichen Zeitpunkten zu messen. Kennzeichnend für den erfindungsgemäßen Straßenfertiger ist, dass die Steuereinheit dazu ausgebildet ist, zu erkennen, dass beim Verbau der Einbauschicht die Messstelle zu einem ersten Zeitpunkt von einem Störobjekt bedeckt ist, wenn entgegen einer zu erwartenden materialspezifischen Abkühlung zu einem späteren zweiten Zeitpunkt während des Verbaus an derselben Messstelle ein größerer, insbesondere innerhalb des sollwertigen Temperaturbereichs liegender, Temperaturwert als ein zu dem vorausgehenden ersten Zeitpunkt erfasster Temperaturwert gemessen wird, und ferner dazu konfiguriert ist, anstelle des zum ersten Zeitpunkts erfassten Temperaturwerts einen neuen Temperaturwert der Messstelle im Temperaturfeld zuzuordnen.

Der erfindungsgemäße Straßenfertiger ist deshalb dazu konfiguriert, rückwirkend für nicht plausibel gehaltene Temperaturmessverläufe, d.h. Temperaturmessungen, welche störobjektbezogen an einer Messstelle der Einbauschicht einen in Abhängigkeit der Zeit ansteigenden Temperaturverlauf anzeigen, anhand eines Steuervorgangs störobjektbezogene Temperaturmessungen zu korrigieren, sodass diese nicht innerhalb des zu erzeugenden Temperaturfelds abgebildet bleiben. Demzufolge kann insgesamt ein präziseres Temperaturfeld, insbesondere für Qualitätskontrollzwecke, hergestellt werden.

Besonders präzise lassen sich nicht den tatsächlichen Temperaturwert einer Messstelle wiedergebende, störobjektbezogene erfasste Temperaturwerte dadurch korrigieren, wenn die Steuereinheit dazu ausgebildet ist, bei der Erstellung des Temperaturfelds für die Einbauschicht als neuen Temperaturwert für die Messstelle den späteren, an derselben Messstelle erfassten größeren Temperaturwert, insbesondere unter Einbeziehung eines ermittelten Abkühlkoeffizienten mindestens einer benachbarten Messstelle, einzusetzen.

Damit in der Einbauschicht zu kalt eingebautes Material im Temperaturfeld zuverlässig angezeigt werden kann, ist die Steuereinheit vorzugsweise dazu ausgebildet, bei der Erstellung des Temperaturfelds für die Einbauschicht den zum ersten Zeitpunkt erfassten Temperaturwert durch den neuen Temperaturwert zu ersetzen, wenn der zum ersten Zeitpunkt erfasste Temperaturwert einen Temperaturschwellenwert nicht übersteigt. Somit kommen für eine Temperaturmessfeldkorrektur im Sinne der Erfindung tatsächlich nur Messstellen entlang der Einbauschicht in Frage, an welchen Temperaturen temporär positionierter Störobjekte erfasst wurden, wobei tatsächliche Einbaumaterialfehler, an Stellen der Einbauschicht, wo Temperaturen oberhalb des Temperaturschwellenwerts und unterhalb des sollwertigen Temperaturbereichs erfasst werden, im Temperaturfeld abgebildet werden.

Eine vorteilhafte Ausführungsform sieht vor, dass die Erfassungseinheit mindestens eine IR-Kamera aufweist, die Temperaturwerte mehrerer Messstellen entlang mindestens einer quer zur Einbaurichtung verlaufenden Messzeile innerhalb des Messbereichs erfasst.

Eine alternative Ausführungsform sieht vor, dass die Erfassungseinheit mindestens einen Zeilensensor aufweist, der dazu ausgebildet ist, jeweilige Temperaturwerte mehrerer Messstellen entlang mindestens einer quer zur Einbaurichtung verlaufenden Messzeile innerhalb des Messbereichs zu erfassen, wobei der Zeilensensor derart beweglich am Straßenfertiger montiert ist, dass die mindestens eine Messzeile während einer Einbaufahrt in Einbaurichtung zum Erfassen der jeweiligen Temperaturen an den darin angeordneten Messstellen zu unterschiedlichen Zeitpunkten zumindest zeitweise entgegengesetzt zur Einbaurichtung bewegt werden kann.

Die Erfindung wird anhand der Figuren genauer erläutert. Es zeigen:
Fig. 1 einen Straßenfertiger mit einer Erfassungseinheit zum Erstellen eines Temperaturfelds mindestens eines Abschnitts einer neu verlegten Einbauschicht hinter der Einbaubohle,
Fig. 2a ein Temperaturfeld einer neuen Einbauschicht inklusive Störobjektfehlmessungen,
Fig. 2b ein erfindungsgemäß um störobjektbezogene Fehlmessungen bereinigtes Temperaturfeld einer neuen Einbauschicht,
Fig. 3a ein schematisch dargestelltes Temperaturfeld mit erfassten Störobjekten,
Fig. 3b ein schematisch dargestelltes Temperaturfeld mit den jeweiligen Fehlmessungen zugeordneten Störobjekten,
Fig. 4 eine schematische Darstellung eines beweglich am Straßenfertiger angeordneten Zeilensensors bzw. eines Flächensensors zur Erfassung des Temperaturfelds der Einbauschicht,
Fig. 5 eine schematisch dargestellter Line-Scanablauf zur Herstellung der Messstellentemperatur zu unterschiedlichen Zeitpunkten,
Fig. 6 eine schematische Darstellung eines Temperaturgradientenverlaufs an einer Messstelle als Hinweis auf ein Störobjekt und
Fig.7 eine schematische Darstellung des erfindungsgemäßen Verfahrens.

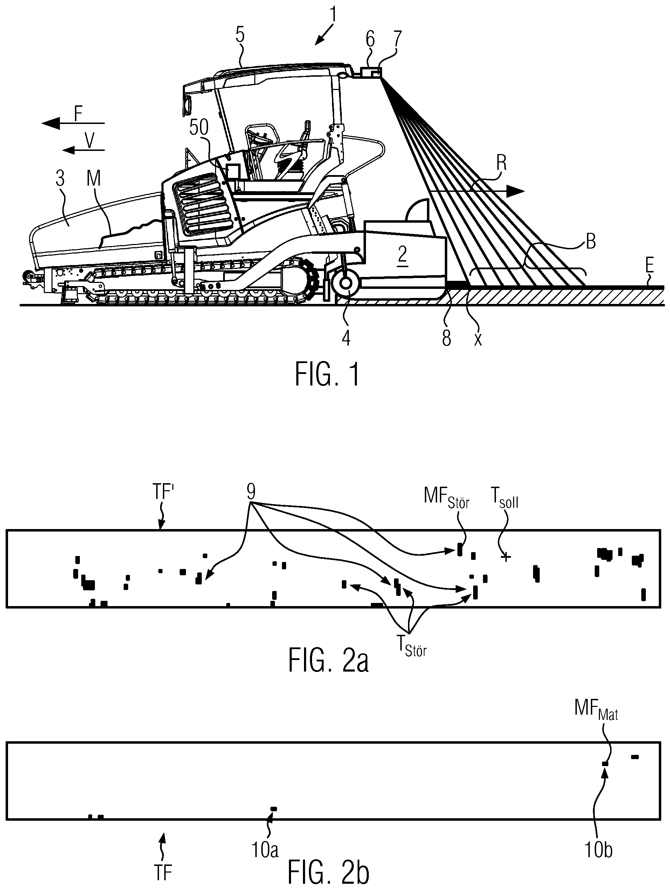

Figur 1 zeigt einen Straßenfertiger 1 zum Herstellen einer Einbauschicht E. Der Straßenfertiger 1 weist eine Einbaubohle 2 auf, welcher aus einem Gutbunker 3 heraus Einbaumaterial M mittels einer nicht gezeigten Längstransportvorrichtung zum Einbau der Einbauschicht E zur Verfügung gestellt wird. Vor der Einbaubohle 2 wird das zu ihr transportiere Einbaumaterial M entsprechend einer Einbaubreite mittels einer Verteilerschnecke 4 verteilt.

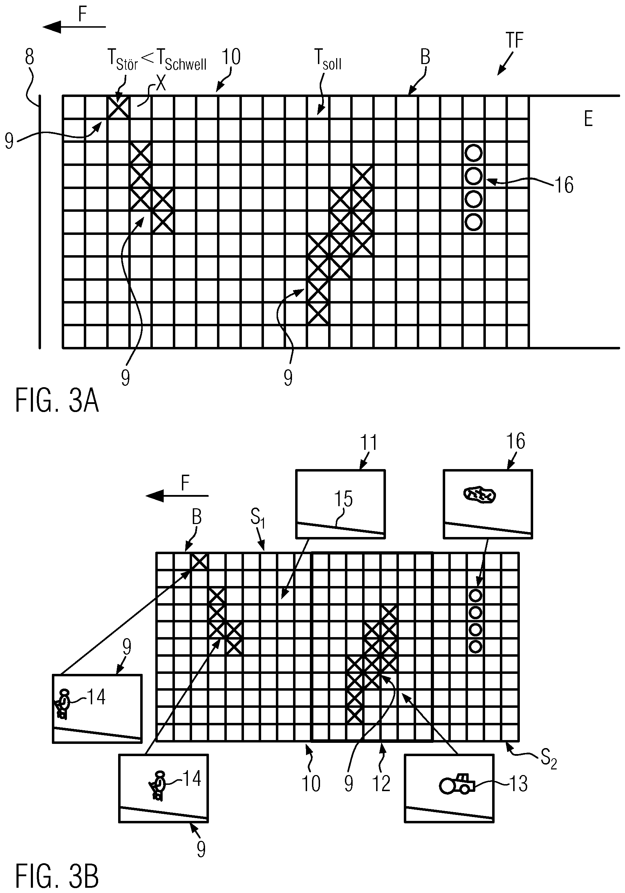

Am Dach 5 des Straßenfertigers 1 ist eine Erfassungseinheit 6 zum Messen eines Oberflächentemperaturprofils der Einbauschicht E befestigt. Die Erfassungseinheit 6 verfügt über einen Zeilensensor 7 oder eine IR-Kamera 7', der/die dazu konfiguriert ist, zumindest intervallweise entgegengesetzt zu einer Einbaurichtung F in einer Messrichtung R quer zur Einbaurichtung F die jeweiligen Temperaturen an über die Einbaubreite der Einbauschicht E verteilten Messstellen x zeilenweise zu erfassen. Der Zeilensensor 7 kann dabei kontinuierlich einen Messbereich B zeilenweise abtasten und erkennen, ob darin ausschließlich sollwertige Temperaturwerte T an den im Messbereich B vorliegenden Messstellen x erfasst werden oder nicht. Für die Messung der Temperaturverteilung kann eine Messzeile 10 (siehe Figur 3a) entlang einer vorbestimmten Strecke ausgehend von einer Bohlenhinterkante 8 langsam in der Messrichtung R entgegengesetzt zur Einbaurichtung F wandern, um zeilenweise die Temperaturverteilung im Messbereich B zu erfassen.

Figur 2a zeigt ein Temperaturfeld TF', in welchem störobjektbezogene Messfehler MF_{Stör} abgebildet sind. In Figur 2a wird deshalb im Temperaturfeld TF' an mehreren Stellen auf erfasste Störobjekte 9 hingewiesen, dessen Eigentemperatur T_{Stör} unterhalb eines gewünschten, sollwertigen Temperaturbereichs Tₛₒₗₗ für die Messstelle x liegt, sodass die Abbildung der Eigentemperatur T_{Stör} der entlang der Einbauschicht E positionierten und erfassten Störobjekte 9 an gleich mehreren Stellen das Temperaturfeld TF' gemäß Figur 2a verfälscht.

Figur 2b zeigt ein hinsichtlich der in Figur 2a gezeigten Störobjekte 9 bereinigtes Temperaturfeld TF, welches mittels einer erfindungsgemäßen Mehrfachmessung erzielbar ist. Gemäß Figur 2b ist das Temperaturfeld TF mit einem homogenen Temperaturprofil ausgebildet, wobei an lediglich zwei Messstellen 10a und 10b auf Materialfehlmessungen MF_{Mat} hingedeutet wird, an welchen Messstellen 10a, 10b tatsächlich zu kaltes Einbaumaterial M verbaut wurde. Störobjekte 9 gemäß Figur 2a sind nicht mehr im Temperaturfeld TF enthalten, weil sie mittels des erfindungsgemäßen Verfahrens herausgefiltert bzw. überschrieben wurden, sodass insgesamt die tatsächliche Materialbeschaffenheit der Einbauschicht E in qualitativer Hinsicht besser beurteilt werden kann.

Figur 3a zeigt den Messbereich B des Temperaturfelds TF der Einbauschicht E. Gemäß Figur 3a werden innerhalb des Messbereichs B an den jeweiligen Messstellen x erfasste Temperaturwerte T entlang mehrerer hintereinander quer zur Einbaurichtung F verlaufenden Messzeilen 10 erfasst.

An den mit einem Kreuz X befüllten Messstellen x befinden sich gemäß jeweiligen Momentaufnahmen nach Figur 3a Störobjekte 9, welche jeweils eine Eigentemperatur T_{Stör} außerhalb des sollwertigen Temperaturbereichs Tₛₒₗₗ haben und ferner einen Temperaturschwellenwert T_{Schwelle} unterschreiten. Bei den mit einem Kreis O in Figur 3a kenntlich gemachten Messstellen x liegt ein tatsächlicher Einbaufehler 16 vor. An diesen Messstellen x wurde zu kaltes Einbaumaterial M verbaut.

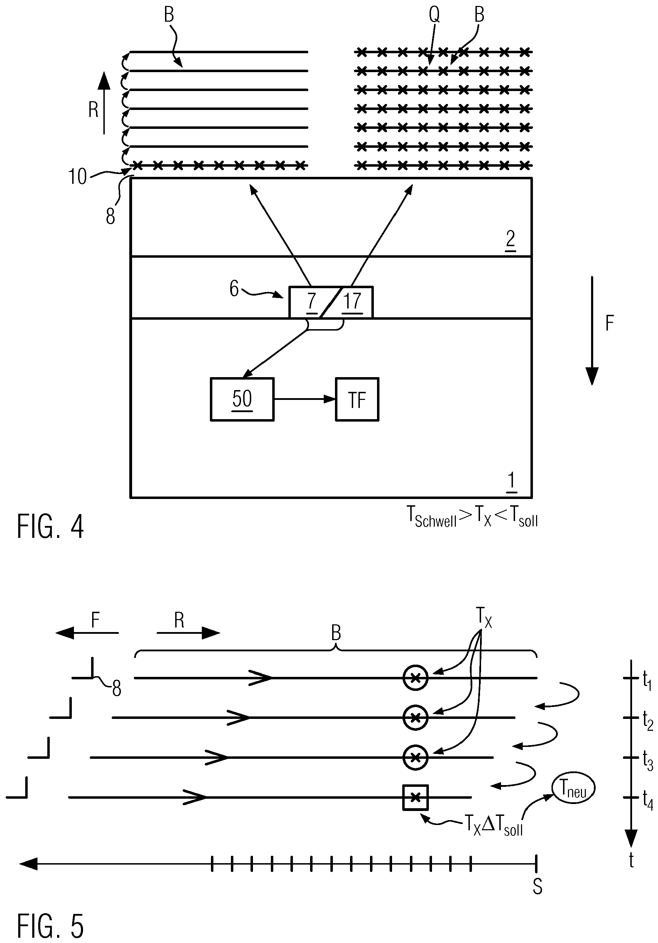

Störobjekte 9 können erfindungsgemäß dadurch detektiert werden, wenn entgegen einer zu erwarteten materialspezifischen Abkühlung 11 (siehe Figur 3b) zu einem späteren Zeitpunkt tₓ (siehe Figur 5) während des Verbaus an derselben Messstelle x ein größerer, insbesondere innerhalb des sollwertigen Temperaturbereichs Tₛₒₗₗ liegenderTemperaturwert T als ein zu einem vorausgehenden Zeitpunkt tₓ₋₁ an derselben Messstelle x erfasster Temperaturwert T gemessen wird, vorzugsweise unter der Maßgabe, dass die zunächst unterhalb des sollwertigen Temperaturbereichs Tₛₒₗₗ erfasste Temperatur T einen Temperaturschwellenwert T_{Schwelle} nicht übersteigt.

Figur 3b zeigt in schematischer Darstellung mögliche Störobjekte 9 entlang des Messbereichs B des Temperaturfelds TF. In einem dick eingerahmten Bereich 12 des Messbereichs B werden an zahlreichen Messstellen x dreier hintereinanderliegender Messzeilen 10 außerhalb des Temperatursollbereichs Tₛₒₗₗ Temperaturen T erfasst, welche gemäß Figur 3b auf ein dem Straßenfertiger 1 hinterherfahrendes Verdichterfahrzeug 13 hinweisen, welches sich im Zeitraum einer dort stattfindenden Temperaturmessung auf der Einbauschicht E bewegt. In Einbaurichtung F an weiter vorderer Stelle des Messbereichs B werden Personen 14 auf der Einbauschicht E erfasst. Im Messbereich B zwischen den erfassten Personen 14 und dem im Bereich 12 erfassten Verdichterfahrzeug 13 liegt in vier hintereinander folgenden Messzeilen 10 an den jeweiligen Messstellen x eines Messsegments S1 eine homogene Temperaturverteilung gemäß dem gewünschten, sollwertigen Temperaturbereich Tₛₒₗₗ vor. Im Messsegment S1 des Messbereichs B wird eine materialspezifische Abkühlung 11, die einen Abkühlkoeffizienten 15 des Einbaumaterials M widergibt, bestimmt.

In einem hinteren Messsegment S2 des Messbereichs B werden entlang der drittletzten Messzeile 10 an vier nebeneinander angeordneten Messstellen x Einbaufehler 16 erfasst, indem an diesen Messstellen x eine Materialtemperatur erfasst wird, die zwar unterhalb des gewünschten, sollwertigen Temperaturbereichs Tₛₒₗₗ, allerdings oberhalb des Temperaturschwellenwerts Tschwelle liegt. Entgegen den störobjektbezogenen Messfehlern MF_{Stör} deuten Materialfehlmessungen MF_{Mat} auf tatsächliche Einbaufehler in der Einbauschicht E hin und gehen folgerichtig in der Herstellung des Temperaturfeldabbilds TF mit ein (siehe Figur 2b).

Figur 4 zeigt in schematischer Darstellung den Straßenfertiger 1 mit der Einbaubohle 2 aus der Draufsicht. Figur 4 zeigt als Variante den Straßenfertiger 1 mit einem Zeilensensor 7 und als alternative Variante den Straßenfertiger 1 mit einem Flächensensor 17. Gemäß Figur 4 ist also am Straßenfertiger 1 entweder ein Zeilensensor 7 oder ein Flächensensor 17 vorgesehen, um die Temperaturverteilung TF der Einbauschicht E zu erfassen.

Der Zeilensensor 7 erfasst während des Einbaus der Einbauschicht E hinter der Einbaubohle 2 jeweilige Temperaturen T an den Messstellen x einer Messzeile 10, die während des Einbaus langsam entgegengesetzt zur Einbaurichtung F in Messrichtung R weg von der Bohlenhinterkante 8 bewegt wird. Das in mehreren wiederkehrenden Intervallen entgegengesetzt zur Einbaurichtung F zeilenweise nach hinten Bewegen der Messzeile 10 ermöglicht es, dass die jeweiligen Temperaturen T mehrerer entlang der Messzeile 10 liegender Messstellen x mehrfach abgetastet werden können, wodurch sich Störobjekte 9 innerhalb des Messbereichs B detektieren lassen.

Mittels des in Figur 4 schematisch dargestellten Flächensensors 17 lassen sich die jeweiligen im Messbereich B an den verschiedenen Messstellen x vorkommenden Temperaturen T flächig, d.h. entlang mehrerer Messzeilen 10 gleichzeitig erfassen. Die vom Flächensensor 17 aufgespannte zweidimensionale Messfläche Q entspricht gemäß Figur 4 dem gesamten Messbereich B, kann aber auch kleiner bestimmt sein.

Die jeweiligen von der Erfassungseinheit 6 erfassten Temperaturwerte T werden einer Steuereinheit 50 des Straßenfertigers 1 zugeführt. Die Steuereinheit 50 ist dazu konfiguriert, das Temperaturfeld TF gemäß Figur 2b zu erzeugen, welches um die störobjektbezogenen Temperaturmessungen MF_{Stör} korrigiert ist.

Figur 5 zeigt in schematischer Darstellung eine zeitliche Abfolge der Messung einer Temperatur Tₓ an einer Messstelle x zu verschiedenen Zeitpunkten t₁ bis t₄ entlang der Einbaustrecke S.

Zu den Zeitpunkten t₁ bis t₃ wird anhand der jeweils eingekreist dargestellten Messstelle x angedeutet, dass die an der Messstelle x während des Einbaus erfassten Temperaturwerte Tₓ zu einem Störobjekt 9 gehören, weil die jeweiligen Temperaturwerte Tx an der Messstelle x kleiner wie der gewünschte, sollwertige Temperaturbereich T_{Soll} und auch kleiner als ein Temperaturschwellenwert T_{Schwelle} sind.

Zum Zeitpunkt t₄ hat das Störobjekt 9 die Messstelle x verlassen, sodass, wie es mit einem Kästchen an der Messstelle x angedeutet wird, nun ein Temperaturwert Tₓ vorliegt, der sollwertig ist. Sofern im Temperaturfeld TF die Eigentemperatur T_{Stör} des Störobjekts 9 abgebildet worden ist, kann nun die Messstelle x mit einem neuen Temperaturwert Tₙₑᵤ belegt werden, welcher dem Temperaturwert Tₓ zurzeit t₄ entspricht, ggf. anhand des ermittelten Abkühlkoeffizienten 15 (siehe Figur 3b) mindestens einer benachbarten Messstelle x korrigiert wird. Der neue Temperaturwert Tₙₑᵤ wird dann verwendet, um den ursprünglichen störobjektbezogenen Temperaturwert Tₓ im Temperaturfeld TF zu überschreiben.

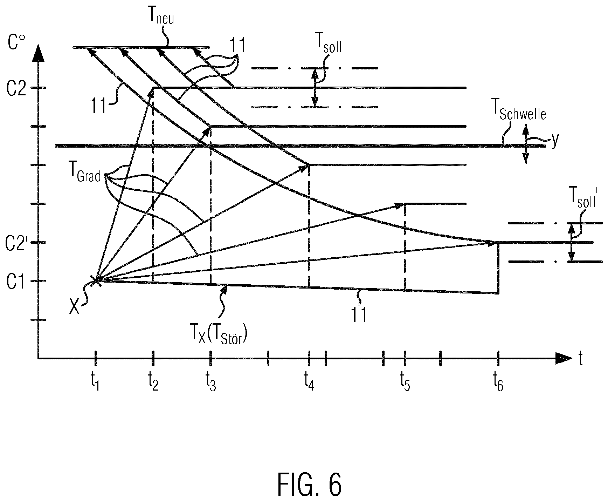

Figur 6 stellt schematisch den erfassten Temperaturwert Tₓ an einer vorbestimmten Messstelle x über der Zeit t dar. Zum Zeitpunkt t1 wird eine Temperatur C1 an der Messstelle x erfasst. Die Temperatur C1 liegt außerhalb des sollwertigen Temperaturbereichs T_{Soll} und unterhalb des Temperaturschwellenwerts T_{Schwelle}. Ferner zeigt Figur 6, dass der Temperaturwert Tₓ aufgrund einer materialspezifischen Abkühlung 11 über die Zeit t1 bis t6 abnimmt.

Ferner zeigt Figur 6 mehrere Temperaturgradienten T_{Grad}, die allgemein eine Störobjekterfassung anzeigen. Beispielsweise wird die zum Zeitpunkt t1 vom Störobjekt 9 bedeckte Messstelle x zum Zeitpunkt t2 freigegeben, d.h. das Störobjekt 9 bedeckt nun zum Zeitpunkt t2 nicht mehr die Messstelle x, sodass zum Zeitpunkt t2 nicht mehr die zum Zeitpunkt t1 erfasste Eigentemperatur T_{Stör}(C1), sondern die Temperatur C2 an der Messstelle x erfasst wird. Die Temperatur C2 liegt gemäß Figur 6 im sollwertigen Temperaturbereich _{TSoll} und kann, vorzugsweise unter Berücksichtigung des Abkühlkoeffizienten 11 einer benachbarten Messstelle x oder mithilfe eines vorbestimmten materialspezifischen Abkühlkoeffizienten, in einen neuen Temperaturwert Tₙₑᵤ umgerechnet werden, der dann mittels der Steuerung 50 im Temperaturfeld TF an der Messstelle x eingesetzt wird.

Sofern das Störobjekt 9 längere Zeit an der Messstelle x verweilt, beispielsweise bis zum Zeitpunkt t6, wird gemäß Figur 6 beim Freigeben der Messstelle x die Temperatur C2' erfasst, die innerhalb eines in Abhängigkeit der Zeit angepassten sollwertigen Temperaturbereichs T_{Soll'} liegt. Auch zum Zeitpunkt t6 kann mithilfe des Abkühlkoeffizienten 11 gemäß Figur 6 der neue Temperaturwert Tₙₑᵤ hergeleitet werden, sodass selbst nach langem Verweilen des Störobjekts 9 an der Messstelle x der neue Temperaturwert Tₙₑᵤ für die Messstelle x im Temperaturfeld TF eingesetzt wird.

Gemäß Figur 6 kann der Temperaturschwellenwert T_{Schwelle} anpassbar (Verlagerung y) sein, vorzugsweise hinsichtlich der verstrichenen Zeit t. Dies kann beispielsweise proportional zu einer Anpassung des sollwertigen Temperaturbereichs T_{Soll} geschehen.

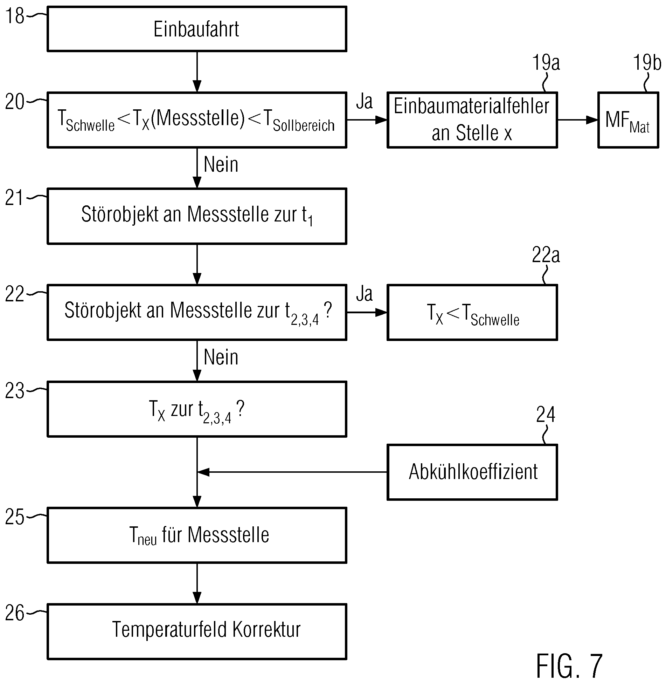

Figur 7 zeigt in schematischer Darstellung ein Verfahren gemäß der Erfindung. Gemäß einem ersten Verfahrensschritt 18 befindet sich der Straßenfertiger 1 in einer Einbaufahrt. Während der Einbaufahrt baut der Straßenfertiger 1 die Einbauschicht E ein. Dabei bewegt sich der Straßenfertiger 1 langsam vorwärts in Einbaurichtung F, wobei die Messzeile 10 langsam von der Bohlenhinterkante 8 weg nach hinten in Messrichtung R wandert, um eine Mehrfachmessung der Temperaturverteilung des Messbereichs B durchzuführen. Alternativ dazu ist eine Flächenmessung gemäß Figur 4 möglich.

An innerhalb des Messbereichs B liegenden Messstellen x werden nun anhand der folgenden Verfahrensschritte mehrfach Temperaturmessungen durchgeführt, um herauszufinden, ob Messstellen x des Messbereichs B von Störobjekten 9 temporär besetzt sind, damit bejahendenfalls das Temperaturfeld TF dementsprechend korrigiert werden kann.

Sofern gemäß dem Verfahrensschritt 20 mittels der Temperaturmessung Temperaturwerte T unterhalb eines gewünschten, sollwertigen Temperaturbereichs Tₛₒₗₗ allerdings oberhalb eines Temperaturschwellenwerts T_{schwelle} erfasst werden, wird im Verfahrensschritt 19a bestimmt, dass Einbaumaterialfehler an diesbezüglichen Messstellen x vorliegen, die gemäß Verfahrensschritt 19b im Temperaturfeld TF abgebildet werden. Wenn jedoch die erfassten Temperaturen nicht sollwertig sind und unterhalb des Temperaturschwellenwerts T_{schwelle} liegen, wird gemäß dem Verfahrensschritt 21 bestimmt, dass an den dazugehörigen Messstellen x ein Störobjekt 9 positioniert ist.

Wird zu einem späteren Zeitpunkt t_{2,3,4} im Schritt 22 erfasst, dass die störobjektbezogene Temperatur T_{Stör} (Schritt 22a) nicht mehr vorliegt, sondern entgegen einer materialspezifischen Abkühlung 11 nun eine größere Temperatur Tₓ an der Messstelle x (Schritt 23) gegeben ist, wird daraus hergeleitet, dass das Störobjekt 9 nicht mehr an der Messstelle x positioniert ist.

Es wird dann die aktuelle Temperatur Tₓ der Messstelle x zum Zeitpunkt t_{2,3,4} erfasst und ggf. mittels des Abkühlkoeffizienten 11 berichtigt (Schritt 24) im Temperaturfeld TF als neue Temperatur Tₙₑᵤ (Schritt 25) für die Messstelle x eingesetzt (Schritt 26).

## Patentansprüche

1. Verfahren zum Erstellen eines Temperaturfelds (TF) mindestens eines Abschnitts einer mittels einer Einbaubohle (2) eines Straßenfertigers (1) verlegten Einbauschicht (E), wobei ein temporär auf der verlegten Einbauschicht (E), innerhalb mindestens eines darauf vorbestimmten Messbereichs (B) des Temperaturfelds (TF) positioniertes Störobjekt (9) detektiert wird, dessen Eigentemperatur (T_{stör}) unterhalb eines gewünschten, sollwertigen Temperaturbereichs (Tₛₒₗₗ) für den Messbereich (B) liegt, sodass eine Abbildung der Eigentemperatur (T_{stör}) des Störobjekts (9) im zu erzeugenden Temperaturfeld (TF) dasselbe zumindest stellenweise verfälscht, wobei zum Detektieren des Störobjekts (9) eine Temperatur (Tₓ) mindestens einer innerhalb des Messbereichs (B) liegenden Messstelle (x) zu unterschiedlichen Zeitpunkten (t₁, t₂) gemessen wird, **dadurch gekennzeichnet, dass** erkannt wird, dass beim Verbau der Einbauschicht (E) die Messstelle (x) zu einem ersten Zeitpunkt (t₁) von einem Störobjekt (9) bedeckt wird, wenn entgegen einer zu erwartenden materialspezifischen Abkühlung (11) zu einem späteren zweiten Zeitpunkt (t₂) während des Verbaus an derselben Messstelle (x) ein größerer, insbesondere innerhalb des sollwertigen Temperaturbereichs (Tₛₒₗₗ) liegender, Temperaturwert (Tₓ) als ein zu dem vorausgehenden ersten Zeitpunkt (t₁) erfasster Temperaturwert (Tₓ) gemessen wird, wobei anstelle des zum ersten Zeitpunkts (t₁) erfassten Temperaturwerts (Tₓ) ein neuer Temperaturwert (Tₙₑᵤ) der Messstelle (x) im Temperaturfeld (TF) zugeordnet wird.

2. Verfahren nach Anspruch 1, **dadurch gekennzeichnet, dass** zum Detektieren des Störobjekts (9) eine Objekterkennung in einer Auswertungssoftware zum Einsatz kommt.

3. Verfahren nach Anspruch 2, **dadurch gekennzeichnet, dass** zum Detektieren des Störobjekts (9) in der Auswertungssoftware überprüft wird ob sich Störobjekte relativ zur Einbaugeschwindigkeit bewegen.

4. Verfahren nach einem der vorangehenden Ansprüche, **dadurch gekennzeichnet, dass** bei der Erstellung des Temperaturfelds (TF) für die Einbauschicht (E) als neuer Temperaturwert (Tₙₑᵤ) für die Messstelle (x) der spätere, an derselben Messstelle (x) erfasste größere Temperaturwert (Tₓ) eingesetzt wird.

5. Verfahren nach Anspruch 4, **dadurch gekennzeichnet, dass** der spätere an derselben Messstelle (x) erfasste größere Temperaturwert (Tₓ) noch unter Berücksichtigung eines ermittelten Abkühlkoeffizienten (15) mindestens einer benachbarten Messstelle (x) korrigiert wird, bevor der dadurch erhaltene neue Temperaturwert (Tₙₑᵤ) den zum ersten Zeitpunkt (t₁) erfassten Temperaturwert (Tₓ) ersetzt.

6. Verfahren nach einem der vorangehenden Ansprüche, **dadurch gekennzeichnet, dass** bei der Erstellung des Temperaturfelds (TF) für die Einbauschicht der zum ersten Zeitpunkt (t₁) erfasste Temperaturwert (Tₓ) durch den neuen Temperaturwert (Tₙₑᵤ) ersetzt wird, wenn der zum ersten Zeitpunkt (t₁) erfasste Temperaturwert (Tₓ) einen Temperaturschwellenwert (T_{schwelle}) nicht übersteigt.

7. Verfahren nach einem der vorangehenden Ansprüche, **dadurch gekennzeichnet, dass** jeweilige Temperaturwerte (Tₓ) mehrerer Messstellen (x) entlang mindestens einer quer zur Einbaurichtung (F) verlaufenden Messzeile (10) innerhalb des Messbereichs erfasst werden.

8. Verfahren nach Anspruch 7, **dadurch gekennzeichnet, dass** die mindestens eine Messzeile (10) während des Verbaus der Einbauschicht (E) zum Erfassen der jeweiligen Temperaturen (Tₓ) an darin angeordneten Messstellen (x) zu unterschiedlichen Zeitpunkten zumindest zeitweise entgegengesetzt zur Einbaurichtung (F) bewegt wird.

9. Verfahren nach Anspruch 8, **dadurch gekennzeichnet, dass** ein Bewegungsablauf mindestens einer Messzeile (10) in Abhängigkeit zu einer Einbaugeschwindigkeit (V) des Straßenfertigers (1) eingestellt wird.

10. Verfahren nach Anspruch 7, **dadurch gekennzeichnet, dass** entlang einer Vielzahl von hintereinander quer zur Einbaurichtung (F) jeweils in einem vorbestimmten Abstand (A) relativ zu einer Bohlenhinterkante (8) der Einbaubohle (2) angeordneter Messzeilen (10) die jeweiligen Temperaturen (Tₓ) der darin angeordneten Messstellen (x) gleichzeitig während des Verbaus der Einbauschicht (E) erfasst werden.

11. Verfahren nach einem der vorangehenden Ansprüche 7 bis 10, **dadurch gekennzeichnet, dass** eine jeweilige Messzeilenbreite (y) in Abhängigkeit einer Einbaubreite der Einbauschicht (E) angepasst wird.

12. Verfahren nach einem der vorangehenden Ansprüche, **dadurch gekennzeichnet, dass** der sollwertige Temperaturbereich (Tₛₒₗₗ) an der Messstelle (x) für unterschiedliche Zeitpunkte (t) unter Berücksichtigung eines ermittelten Abkühlkoeffizienten (15) mindestens einer benachbarten Messstelle (x) angepasst wird.

13. Verfahren nach einem der vorangehenden Ansprüche, **dadurch gekennzeichnet, dass**, wenn ein Störobjekt (9) erkannt wurde, am Straßenfertiger (1), am Störobjekt (9) selbst und/oder an einem anderen am Einbau beteiligten Fahrzeug (13) eine visuelle, akustische und/oder haptische Signalgebung einsetzt.

14. Straßenfertiger (1), umfassend eine Einbaubohle (2) zum Verlegen einer Einbauschicht (E), eine Erfassungseinheit (6) zum Erstellen eines Temperaturfelds (TF) mindestens eines Abschnitts der Einbauschicht (E) im verlegten Zustand hinter der Einbaubohle (2) sowie eine Steuereinheit (50), die dazu konfiguriert ist, basierend auf Messwerten (Tₓ) der Erfassungseinheit (6) ein temporär auf der Einbauschicht (E), innerhalb mindestens eines darauf vorbestimmten Messbereichs (B) des Temperaturfelds (TF) positioniertes Störobjekt (9) zu detektieren, dessen Eigentemperatur (T_{stör}) unterhalb eines gewünschten, sollwertigen Temperaturbereichs (Tₛₒₗl) für den Messbereich (B) liegt, sodass eine Abbildung der Eigentemperatur (T_{stör}) des Störobjekts (9) im zu erzeugenden Temperaturfeld (TF) dasselbe zumindest stellenweise verfälscht, wobei die Erfassungseinheit (6) dazu ausgebildet ist, zum Detektieren des Störobjekts (9) eine Temperatur (Tₓ) mindestens einer innerhalb des Messbereichs (B) liegenden Messstelle (x) zu unterschiedlichen Zeitpunkten (t) zu messen, **dadurch gekennzeichnet, dass** die Steuereinheit (50) dazu ausgebildet ist, zu erkennen, dass beim Verbau der Einbauschicht (E) die Messstelle (x) zu einem ersten Zeitpunkt (t₁) von einem Störobjekt (9) bedeckt ist, wenn entgegen einer zu erwartenden materialspezifischen Abkühlung (11) zu einem späteren zweiten Zeitpunkt (t2) während des Verbaus an derselben Messstelle (x) ein größerer, insbesondere innerhalb des sollwertigen Temperaturbereichs (B) liegender, Temperaturwert (Tₓ) als ein zu dem vorausgehenden ersten Zeitpunkt (t₁) erfasster Temperaturwert (Tₓ) gemessen wird, und ferner dazu konfiguriert ist, anstelle des zum ersten Zeitpunkts (t₁) erfassten Temperaturwerts (Tₓ) einen neuen Temperaturwert (Tₙₑᵤ) der Messstelle (x) im Temperaturfeld (TF) zuzuordnen.

15. Straßenfertiger nach Anspruch 14, **dadurch gekennzeichnet, dass** die Steuereinheit (50) dazu ausgebildet ist, bei der Erstellung des Temperaturfelds (TF) für die Einbauschicht als neuen Temperaturwert (Tₙₑᵤ) für die Messstelle (x) den späteren, an derselben Messstelle (x) erfassten größeren Temperaturwert (Tₓ), insbesondere unter Einbeziehung eines ermittelten Abkühlkoeffizienten (15) mindestens einer benachbarten Messstelle (x), einzusetzen.

16. Straßenfertiger nach Anspruch 14 oder 15, **dadurch gekennzeichnet, dass** die Steuereinheit (50) dazu ausgebildet ist, bei der Erstellung des Temperaturfelds (TF) für die Einbauschicht (E) den zum ersten Zeitpunkt (t₁) erfassten Temperaturwert (Tₓ) durch den neuen Temperaturwert (Tₙₑᵤ) zu ersetzen, wenn der zum ersten Zeitpunkt (t₁) erfasste Temperaturwert (Tₓ) einen Temperaturschwellenwert (T_{schwelle}) nicht übersteigt.

17. Straßenfertiger nach einem der vorangehenden Ansprüche 14 bis 16, **dadurch gekennzeichnet, dass** die Erfassungseinheit (6) mindestens einen Zeilensensor (7) aufweist, der dazu ausgebildet ist, jeweilige Temperaturwerte (Tₓ) mehrerer Messstellen (x) entlang mindestens einer quer zur Einbaurichtung (E) verlaufenden Messzeile (10) innerhalb des Messbereichs (B) zu erfassen, wobei der Zeilensensor (7) derart beweglich am Straßenfertiger (1) montiert ist, dass die mindestens eine Messzeile (10) während einer Einbaufahrt in Einbaurichtung (F) zum Erfassen der jeweiligen Temperaturen (Tₓ) an den darin angeordneten Messstellen (x) zu unterschiedlichen Zeitpunkten (t) zumindest zeitweise entgegengesetzt zur Einbaurichtung (F) bewegt werden kann.

18. Straßenfertiger nach einem der vorangehenden Ansprüche 14 bis 16, **dadurch gekennzeichnet, dass** die Erfassungseinheit mindestens eine Infrarotkamera (7') aufweist, die Temperaturwerte (Tₓ) mehrere Messstellen (x) entlang mindestens einer quer zur Einbaurichtung (E) verlaufenden Messzeile (10) innerhalb des Messbereichs (B) erfasst.

## Claims

1. Method for creating a temperature field (TF) of at least one section of a paving layer (E) laid by means of a paving screed (2) of a road finisher (1), an interfering object (9) temporarily positioned on the laid paving layer (E) within at least one predetermined measuring range (B) of the temperature field (TF) being detected, the intrinsic temperature (T_{interf}) of which being below a desired nominal temperature (Tₙₒₘ) for the measuring range (B), so that an imaging of the intrinsic temperature (T_{interf}) of the interfering object (9) in the temperature field (TF) to be generated falsifies the same at least in places, wherein for detecting the interfering object (9) a temperature (Tₓ) of at least one measuring point (x) lying within the measuring range (B) is measured at different times (t₁, t₂), **characterized in that** it is detected that during the paving of the paving layer (E), the measuring point (x) is covered by a interfering object (9) at a first time (t₁) if, contrary to an expected material-specific cooling (11) at a later second time (t₂) during the paving, at the same measuring point (x) a larger temperature value (Tₓ) particularly lying within the nominal temperature range (Tₙₒₘ) than a temperature value (Tₓ) detected with respect to the preceding first time (t₁) is measured, wherein instead of the temperature value (Tₓ) detected at the first time (t1) a new temperature value (T_{new}) of the measuring point (x) is assigned in the temperature field (TF).

2. Method according to claim 1, **characterized in that** for detecting the interfering object (9) an object detection in an evaluation software is used.

3. Method according to claim 2, **characterized in that** for detecting the interfering object (9) it is checked in the evaluation software whether interfering objects move relative to the paving speed.

4. Method according to one of the preceding claims, **characterized in that,** when creating the temperature field (TF) for the paving layer (E), the later larger temperature value (Tₓ) detected at the same measuring point (x) is used as the new temperature value (T_{new}) for the measuring point (x).

5. Method according to claim 4, **characterized in that** the later greater temperature value (Tₓ) detected at the same measuring point (x) is corrected taking into account a determined cooling coefficient (15) of at least one adjacent measuring point (x) before the new temperature value (T_{new}) thus obtained replaces the temperature value (Tₓ) detected at the first time (t₁).

6. Method according to one of the preceding claims, **characterized in that,** when the temperature field (TF) is created for the paving layer, the temperature value (Tₓ) detected at the first time (t1) is replaced by the new temperature value (T_{new}) if the temperature value (Tₓ) detected at the first time (t₁) does not exceed a temperature threshold value (Tₜₕᵣₑₛ).

7. Method according to one of the preceding claims, **characterized in that** respective temperature values (Tₓ) of a plurality of measuring points (x) are detected along at least one measuring line (10) running transversely to the paving direction (F) within the measuring range.

8. Method according to claim 7, **characterized in that** the at least one measuring line (10) is moved at different times at least temporarily opposite to the paving direction (F) during the paving of the paving layer (E) for detecting the respective temperatures (Tₓ) at measuring points (x) arranged therein.

9. Method according to claim 8, **characterized in that** a motion sequence of at least one measuring line (10) is set as a function of a paving speed (V) of the road finisher (1).

10. Method according to claim 7, **characterized in that** along a plurality of measuring lines (10) arranged one behind the other transversely to the paving direction (F) at a predetermined distance (A) relative to a screed rear edge (8) of the paving screed (2), the respective temperatures (Tₓ) of the measuring points (x) arranged therein are detected simultaneously during the paving of the paving layer (E).

11. Method according to one of the preceding claims 7 to 10, **characterized in that** a respective measuring line width (y) is adapted with respect to a paving width of the paving layer (E).

12. Method according to one of the preceding claims, **characterized in that** the nominal temperature range (Tₙₒₘ) at the measuring point (x) is adapted for different times (t) taking into account a determined cooling coefficient (15) of at least one adjacent measuring point (x).

13. Method according to one of the preceding claims, **characterized in that,** if an interfering object (9) has been detected, a visual, acoustic and/or haptic signal is emitted on the road finisher (1), on the interfering object (9) itself and/or on another vehicle (13) involved in the paving.

14. A road finisher (1) comprising a paving screed (2) for laying a paving layer (E), a detection unit (6) for creating a temperature field (TF) of at least a portion of the paving layer (E) in the laid state behind the paving screed (2), and a control unit (50) which, based on the measured values (Tₓ) of the detection unit (6), is configured to detect an interfering object (9) positioned temporarily on the paving layer (E) within at least one predetermined measuring range (B) of the temperature field (TF), whose intrinsic temperature (T_{interf}) lies below a desired, nominal temperature range (Tₙₒₘ) for the measuring range (B) so that a mapping of the intrinsic temperature (T_{interf}) of the interfering object (9) at least in places falsifies the temperature field (TF) to be generated, wherein the detection unit (6) is adapted for detecting the interfering object (9) to measure a temperature (Tx) of at least one measuring point (x) lying within the measuring range (B) at different times (t), **characterized in that** the control unit (50) is configured to detect that, when the paving layer (E) is paved, the measuring point (x) is covered by an interfering object (9) at a first time (t₁), if, contrary to an expected material-specific cooling (11) at a later second time (t₂) during paving at the same measuring point (x), a larger temperature value (Tₓ) particularly lying within the nominal temperature range (Tₙₒₘ) than a temperature value (Tₓ) measured at the preceding first time (t1) is measured, and is further configured to assign a new temperature value (T_{new}) to the measuring point (x) in the temperature field (TF) instead of the temperature value (Tₓ) detected at the first time (t₁).

15. Road finisher according to claim 14, **characterized in that** the control unit (50) is configured, when the temperature field (TF) for the paving layer is created, to use, as a new temperature value (T_{new}) for the measuring point (x), the later larger temperature value (Tₓ) recorded at the same measuring point (x), in particular with inclusion of a determined cooling coefficient (15) of at least one adjacent measuring point (x).

16. Road finisher according to claim 14 or 15, **characterized in that** the control unit (50) is configured, when the temperature field (TF) for the paving layer (E) is created, to replace the temperature value (Tₓ) detected at the first time (t₁) by the new temperature value (T_{new}), if the temperature value (Tₓ) detected at the first time (t₁) does not exceed a temperature threshold value (Tₜₕᵣₑₛ).

17. Road finisher according to one of the preceding claims 14 to 16, **characterized in that** the detection unit (6) has at least one line sensor (7) which is designed to detect respective temperature values (Tₓ) of a plurality of measuring points (x) along at least one measuring line (10) running transversely to the direction of paving (E) within the measuring range (B), the line sensor (7) being mounted movably on the road finisher (1) in such a way that the at least one measuring line (10) can be moved at different times (t) at least temporarily in the opposite direction to the paving direction (F) during a paving operation for detecting the respective temperatures (Tₓ) at the measuring points (x) arranged therein.

18. Road finisher according to one of the preceding claims 14 to 16, **characterized in that** the detection unit has at least one infrared camera (7') which detects temperature values (Tₓ) at a plurality of measuring points (x) along at least one measuring line (10) running transversely to the direction of paving (E) within the measuring range (B).

## Revendications

1. Procédé de création d'un champ de température (TF) d'au moins une section d'une couche d'ouvrage (E) déposée au moyen d'une table de réglage (2) d'un finisseur (1), dans lequel un objet perturbateur (9), positionné de manière temporaire sur la couche d'ouvrage (E) déposée dans au moins une plage de mesure (B) prédéterminée du champ de température (TF) et dont la température intrinsèque (T_{stör}) est inférieure à une plage de température à valeur de consigne (Tₛₒₗₗ) souhaitée pour la plage de mesure (B), est détecté, de sorte qu'une représentation de la température intrinsèque (T_{stör}) de l'objet perturbateur (9) dans le champ de température (TF) à générer fausse celui-ci au moins par endroits, dans lequel une température (Tₓ) d'au moins un point de mesure (x) situé dans la plage de mesure (B) est mesurée à différents instants (t₁, t₂) afin de détecter l'objet perturbateur (9), **caractérisé en ce que** le fait que le point de mesure (x) est recouvert à un premier instant (t₁) par un objet perturbateur (9) lors de la pose de la couche d'ouvrage (E) est constaté si, contrairement à un refroidissement (11) prévisible spécifique au matériau à un second instant (t₂) ultérieur pendant la pose au niveau du même point de mesure (x), une valeur de température (Tₓ), en particulier située dans la plage de température (Tₛₒₗₗ) à valeur de consigne, supérieure à une valeur de température (Tₓ) enregistrée au premier instant (t₁) précédent est mesurée, dans lequel une nouvelle valeur de température (Tₙₑᵤ) est associée au point de mesure (x) dans le champ de température (TF) à la place de la valeur de température (Tₓ) enregistrée au premier instant (t₁).

2. Procédé selon la revendication 1, **caractérisé en ce qu'**une constatation d'objet au sein d'un logiciel d'évaluation est utilisée afin de détecter l'objet perturbateur (9).

3. Procédé selon la revendication 2, **caractérisé en ce que** le logiciel d'évaluation vérifie si des objets perturbateurs se déplacent par rapport à la vitesse d'ouvrage afin de détecter l'objet perturbateur (9).

4. Procédé selon l'une quelconque des revendications précédentes, **caractérisé en ce que** la température (Tₓ) supérieure ultérieure, enregistrée au niveau du même point de mesure (x), est utilisée en tant que nouvelle valeur de température (Tₙₑᵤ) pour le point de mesure (x) lors de la création du champ de température (TF) pour la couche d'ouvrage (E).

5. Procédé selon la revendication 4, **caractérisé en ce que** la température (Tₓ) supérieure ultérieure enregistrée au niveau du même point de mesure (x) est corrigée en tenant compte d'un coefficient de refroidissement (15) déterminé d'au moins un point de mesure (x) voisin avant que la nouvelle valeur de température (Tₙₑᵤ) ainsi obtenue ne remplace la valeur de température (Tₓ) enregistrée au premier instant (t₁).

6. Procédé selon l'une quelconque des revendications précédentes, **caractérisé en ce que** la valeur de température (Tₓ) enregistrée au premier instant (t₁) est remplacée par la nouvelle valeur de température (Tₙₑᵤ) lors de la création du champ de température (TF) pour la couche d'ouvrage si la valeur de température (Tₓ) enregistrée au premier instant (t₁) ne dépasse pas une valeur de seuil de température (T_{schwelle}).

7. Procédé selon l'une quelconque des revendications précédentes, **caractérisé en ce que** des valeurs de température (Tₓ) respectives de plusieurs points de mesure (x) sont enregistrées dans la plage de mesure le long d'au moins une ligne de mesure (10) s'étendant de manière transversale par rapport au sens d'ouvrage (F).

8. Procédé selon la revendication 7, **caractérisé en ce que** la au moins une ligne de mesure (10) est déplacée de manière au moins temporairement opposée au sens d'ouvrage (F) pendant la pose de la couche d'ouvrage (E) afin d'enregistrer les températures (T_{X}) respectives à différents instants au niveau de points de mesure (x) agencés sur ladite ligne.

9. Procédé selon la revendication 8, **caractérisé en ce qu'**une séquence de déplacement d'au moins une ligne de mesure (10) est réglée en fonction d'une vitesse d'ouvrage (V) du finisseur (1).

10. Procédé selon la revendication 7, **caractérisé en ce que** les températures (Tₓ.) respectives des points de mesure (x) agencés sur des lignes de mesure sont enregistrés de manière simultanée pendant la pose de la couche d'ouvrage (E) le long d'une pluralité desdites lignes de mesure (10) agencées les unes derrière les autres de manière transversale par rapport au sens d'ouvrage avec respectivement une distance (A) prédéterminée par rapport à un bord de fuite (8) de la table de réglage (2).

11. Procédé selon l'une quelconque des revendications 7 à 10, **caractérisé en ce qu'**une largeur de ligne de mesure (y) respective est ajustée en fonction d'une largeur d'ouvrage de la couche d'ouvrage (E).

12. Procédé selon l'une quelconque des revendications précédentes, **caractérisé en ce que** la plage de température à valeur de consigne (Tₛₒₗₗ) au niveau du point de mesure (x) pour différents instants (t) est adaptée en tenant compte d'un coefficient de refroidissement (15) déterminé d'au moins un point de mesure (x) voisin.

13. Procédé selon l'une quelconque des revendications précédentes, **caractérisé en ce que**, si un objet perturbateur (9) a été constaté, une signalisation visuelle, acoustique et/ou haptique est utilisée au niveau du finisseur (1), au niveau de l'objet perturbateur (9) lui-même et/ou au niveau d'un autre véhicule (13) impliqué dans l'ouvrage.

14. Finisseur (1), comprenant une table de réglage (2) destinée à la dépose d'une couche d'ouvrage (E), une unité d'enregistrement (6) destinée à la création d'un champ de température (TF) d'au moins une section de la couche d'ouvrage (E), à l'état déposé, derrière la table de réglage (2) et une unité de commande (50) configurée, sur la base de valeurs de mesure (Tₓ) de l'unité d'enregistrement (6), pour détecter un objet perturbateur (9) positionné de manière temporaire sur la couche d'ouvrage (E) dans au moins une plage de mesure (B) prédéterminée du champ de température (TF) et dont la température intrinsèque (T_{stör}) est inférieure à une plage de température à valeur de consigne (Tₛₒₗₗ) souhaitée pour la plage de mesure (B), de sorte qu'une représentation de la température intrinsèque (T_{stör}) de l'objet perturbateur (9) dans le champ de température (TF) à générer fausse celui-ci au moins par endroits, dans lequel l'unité d'enregistrement (6) est conçue pour mesurer une température (Tₓ) d'au moins un point de mesure (x) situé dans la plage de mesure (B) à différents instants (t) afin de détecter l'objet perturbateur, **caractérisé en ce que** l'unité de commande (50) est conçue pour constater que, lors de la pose de la couche d'ouvrage (E), le point de mesure (x) est recouvert à un premier instant (t₁) par un objet perturbateur (9) si, contrairement à un refroidissement (11) prévisible spécifique au matériau à un second instant (t₂) ultérieur pendant la pose au niveau du même point de mesure (x), une valeur de température (Tₓ), en particulier située dans la plage de température à valeur de consigne (B), supérieure à une valeur de température (Tₓ) enregistrée au premier instant (t₁) précédent, est mesurée, et est en outre configurée pour associer une nouvelle valeur de température (Tₙₑᵤ) au point de mesure (x) dans le champ de température (TF) à la place de la valeur de température (Tₓ) enregistrée au premier instant (t1).

15. Finisseur selon la revendication 14, **caractérisé en ce que** l'unité de commande (50) est conçue pour utiliser la température (Tₓ) supérieure ultérieure, enregistrée au niveau du même point de mesure (x), en tant que nouvelle valeur de température (Tₙₑᵤ) pour le point de mesure (x), en particulier en tenant compte d'un coefficient de refroidissement (15) déterminé d'au moins un point de mesure (x) voisin lors de la création du champ de température (TF) pour la couche d'ouvrage.

16. Finisseur selon la revendication 14 ou 15, **caractérisé en ce que** l'unité de commande (50) est conçue pour, lors de la création du champ de température (TF) pour la couche de revêtement (E), remplacer la valeur de température (Tₓ) enregistrée au premier instant (t₁) par la nouvelle valeur de température (Tₙₑᵤ) si la valeur de température (Tₓ) enregistrée au premier instant (t₁) ne dépasse pas une valeur de seuil de température (T_{schwelle}).

17. Finisseur selon l'une quelconque des revendications 14 à 16, **caractérisé en ce que** l'unité d'enregistrement (6) présente au moins un capteur de ligne (7) conçu pour enregistrer des valeurs de température (Tₓ) respectives de plusieurs points de mesure (x) dans la plage de mesure (B) le long d'au moins une ligne de mesure (10) s'étendant de manière transversale par rapport au sens d'ouvrage (E), dans lequel le capteur de ligne (7) est monté mobile au niveau du finisseur (1) de telle manière que la au moins une ligne de mesure (10) peut être déplacée de manière au moins temporairement opposée au sens d'ouvrage (F) pendant une passe d'ouvrage dans le sens d'ouvrage (F) afin d'enregistrer à différents instants (t) les températures respectives (Tₓ) au niveau des points de mesure (x) agencés sur ladite ligne.

18. Finisseur selon l'une quelconque des revendications 14 à 16, **caractérisé en ce que** l'unité d'enregistrement présente au moins une caméra infrarouge (7') qui enregistre des valeurs de température (Tₓ) de plusieurs points de mesure (x) dans la plage de mesure (B) le long d'au moins une ligne de mesure (10) s'étendant de manière transversale par rapport au sens d'ouvrage (E).
